# EUROPEAN PATENT SPECIFICATION

(11) **EP 4 491 703 B1**
(45) Date of publication and mention of the grant of the patent: **28.01.2026**
(21) Application number: 23185003.3
(22) Date of filing: 12.07.2023
(51) Int. Cl.: C12M 1/00, C12M 1/34

(54) **A SENSOR HEAD AND A METHOD FOR MANUFACTURING A SENSOR HEAD**
SENSORKOPF UND VERFAHREN ZUR HERSTELLUNG EINES SENSORKOPFS
TÊTE DE CAPTEUR ET PROCÉDÉ DE FABRICATION D'UNE TÊTE DE CAPTEUR

(43) Date of publication of application: 15.01.2025
(73) Proprietor: Imec VZW, 3001 Leuven (BE)
(72) Inventor: Gonzalez Losada, Pedro, 3000 Leuven (BE); Paeps, Filip, 3061 Leefdaal (BE); Socolovsky, Alejandro, 3001 Heverlee (BE); Emmen, Erik, 1731 Relegem (BE); Labie, Riet, 3210 Lubbeek (BE); Arnett, Chad, 1090 Jette (BE)
(74) Representative: AWA Sweden AB

(56) References cited:
- EP-A1- 1 972 269
- EP-A1- 3 922 172
- EP-B1- 2 544 809
- WO-A1-2021/181078
- WO-A1-2022/109612
- US-A1- 2005 242 479
- US-A1- 2007 163 353
- US-A1- 2009 281 584
- US-A1- 2012 329 151
- US-A1- 2014 322 701
- US-A1- 2016 298 068
- US-A1- 2017 218 320
- US-B2- 8 568 657

## Description

### Technical field

The present description relates to a sensor head for measurement of a biological material, a probe for insertion into a bioreactor, a sensing device, a bioreactor, a flow cell and a method for manufacturing a sensor head for measurement of a biological material.

### Background

The study of phenomena occurring in biological systems often involves a dependency on more than one parameter. This puts a high demand on a sensor system used for the measurements, as a plurality of sensors may be needed for detecting the parameters. Conventionally, a sensor system having several sensors for enabling multiple parameter analysis may be very bulky putting restrains on usefulness of such sensor systems.

US 2009/281584 discloses an implantable medical sensor arrangement having a sensor body configured for implantation. At least one sensor head, preferably multiple different sensor heads are connected to the sensor body with at least one connective wire.

Many biological environments are liquid environments, further enhancing the difficulties for the use of sensors, since electronics associated with the sensors must be protected from the liquids. The use of multiple dies and protection of electronics may cause sensor devices to be large and bulky. Further, the manufacturing process may be difficult when several sensors are to be provided.

Thus, there is a need in the art for improvements.

### Summary

An objective of the present description is to provide a compact arrangement of sensors for measurement of a biological material. A further objective is to provide a method for manufacturing such a compact arrangement of sensors.

This and other objectives of the present inventive concept are at least partly met by the invention as defined in the independent claims. Preferred embodiments are set out in the dependent claims.
According to a first aspect, there is provided a sensor head for measurement of a biological material, the sensor head comprising:
a substrate comprising:
   a plurality of sensors arranged in a sensor area at a surface of the substrate;
   electrical conductors connecting the plurality of sensors to contacts, wherein the electrical conductors are extending from the sensor area along the surface of the substrate to the contacts;
a cap comprising a protective element for providing protection from the biological material, the protective element comprising:
   a first surface and a second surface, opposite to the first surface;
   walls extending from the first surface to the second surface, defining an opening through the protective element;
wherein the second surface of the protective element, around the walls of the protective element, is sealed to the surface of the substrate at a position between the sensor area and the contacts such that the opening of the protective element is arranged above the sensor area for allowing the plurality of sensors to be exposed to the biological material.

Thanks to the sensor head, several parameters may be measured in the biological material. The sensor head provides a plurality of sensors, which each may be used to analyze a single parameter and together they may be used to analyze a plurality of parameters. However, it should be realized that the plurality of sensors need not necessarily be used for analyzing different parameters. Rather, all sensors may be used for analyzing a same parameter. The use of the plurality of sensors may then, for instance, provide redundancy in performing measurements.

The sensor head may comprise biocompatible materials. The sensor head may at least partly comprise biocompatible materials at surfaces of the sensor head facing the outside environment.

The sensor head may be compatible with common sterilization techniques. In other words, the sensor head may be compatible with sterilization by gamma radiation, autoclavation, steam-in-place sterilization, sterilization by the use of ethylene oxide or nitrogen dioxide.

The biological material may be biological material inside of a bioreactor, inside the body of a human or animal or inside a tubing

The plurality of sensors may measure one or more parameters. The plurality of sensors may comprise at least five sensors, such as at least 10 sensors or at least 20 sensors. In fact, the plurality of sensors may even comprise up to hundreds of sensors. Several sensors may measure the same parameters, such that each parameter has several data points. The plurality of sensors may for instance measure glucose, lactate, electrical conductivity, dissolved oxygen, oxidation-reduction potential and cell density. The sensors may be adapted for measuring physical parameters, chemical parameters or biochemical parameters. Physical parameters may be temperature, conductivity and cell density. Chemical parameters may be pH, ions, dissolved gasses such as oxygen. Biochemical parameters may be protein contents, contaminants, metabolites such as glucose. The combination of sensors may provide with a more complex information of the biological material. The plurality of sensors may be used for one-time measurement or multiple time measurements.

The substrate may be any substrate on which sensors may be formed. The substrate may for instance be a substrate that is compatible with semiconductor processing, such as a silicon substrate, possibly provided with an insulating layer on the silicon substrate. The substrate may alternatively be glass, which may be used as a substrate for forming structures by thin-film deposition on the substrate. The substrate may for instance be a die, which may be formed by semiconductor processing. The substrate has a sensor area, at which the plurality of sensors is arranged.

The plurality of sensors are connected to contacts by electrical conductors. The electrical conductors are extending from the sensor area, along the surface of the substrate and to the contacts. The electrical conductors may conduct electrical signals from the sensors to the contact, such that signals from the sensors are transported from the plurality of sensors to the contact.

The electrical conductors may be any structures allowing electrical signals to be transferred along the electrical conductors. The electrical conductors may for instance be paths, traces or lines formed along the surface of the substrate for conducting signals between the sensors and the contacts. The electrical conductors may be formed on the substrate and may further be protected by a (thin) insulating layer so as not to be exposed to the outside environment.

The contacts provide points to which a wire or any other electrically conducting structure may be connected. Thus, signals conducted by the electrical conductors from the sensors to the contacts may be further transferred from the contacts to a wire or another electrically conducting structure that may be connected to the contact. For instance, the contacts may be bond pads to which wires may be connected.

The sensor head further comprises a cap. The cap comprises a protective element, for providing protection from the biological material. Thanks to the use of the cap comprising the protective elements, the biological material may only reach the sensors and further electronics are protected from the biological material.

The protective element comprises a first surface and a second surface, being opposite to each other. Further, the protective elements comprise walls extending from the first surface to the second surface, defining an opening through the protective element. The walls provide a further protection, such that biological material may not leak through the sides of the protective element.

The second surface of the protective element, around the walls of the protective element, is sealed to the surface of the substrate at a position between the sensor area and the contacts such that the opening of the protective element is arranged above the sensor area for allowing the plurality of sensors to be exposed to the biological material.

The sealing of the second surface of the protective element to the surface further provides the protection of the area of the substrate and the inside of the sensor head from the biological material.

The sealing of the second surface of the protective element to the surface provides an exposition window in which the plurality of sensors may be exposed to the biological material while other components of the sensor head are protected from coming into contact with the biological material. Thus, the protective element protects the contacts of the sensor head from the biological material, while still allowing the biological material to reach the plurality of sensors.

Thanks to the protective element being sealed to the substrate at a position between the sensor area and the contacts, the sensor area may be exposed to the biological material while the electronics may be protected. This also ensures that a plurality of sensors may be used in the sensor area.

Moreover, the sensor head may be sterilized such that only the outside of the sensor head is sterilized. Thanks to the sealing of the protective element to the substrate, sterilization of the inside of the sensor head may not be needed, since the inside is not exposed to the biological material.

The cap may require high precision manufacturing to accurately arrange the cap in relation to the substrate and seal the protective element of the cap to the surface of the substrate. However, with such high precision manufacturing, a sensor head is defined which may be attached to or integrated in a device that is to carry the sensor head without any requirement on high precision attachment. Thus, by using a high precision step for attaching the cap to the substrate, a sensor head is formed which provides lenient requirements on further processing to manufacture a device incorporating the sensor head.

According to one embodiment the cap may further comprise a structure presenting a surface for allowing the cap to be sealed to a cover surrounding the substrate, wherein the protective element may extend over an entire area of the surface of the substrate structure and the structure may be arranged at an outside of the area of the surface of the substrate.

In other words, the protective element may be arranged over the entire substrate, such that the protective element covers the entire substrate. The protective element may be arranged such that a projection of the protective element onto the surface of the substrate covers the entire area of the substrate (except for the exposition window defined by the opening through the protective element). The structure is arranged outside the area of the surface of the substrate such that the projection of the structure onto a plane in which the surface of the substrate is arranged will be outside the surface.

This implies that the structure may be easily accessible for attaching of the cover to the cap. An exact position of the structure to which the cover is to be sealed is not critical from a point of view of function of the sensor head. Therefore, the cap presents a structure that allows a cover to be attached to the cap with lenient requirements on precision in attaching the cover to the cap. Thus, the sensor head may provide a high precision arrangement of a seal around the sensor area while providing lenient requirements on attaching of a cover (which may be large and therefore difficult to attach with very high precision). The sensor head thus enables a seal to be formed at a structure outside an area of the surface of the substrate as well as to the surface of the substrate at a position between the sensor area and the contacts. By this, the cap and the substrate are sealed together such that no biological material may reach the substrate at the place between the sealings. By this, the contacts of the sensor head and other electronics are protected from the outside environment.

According to one embodiment the sensor head may further comprise a printed circuit board, wherein the substrate may be attached to the printed circuit board with the surface of the substrate on which the sensors are arranged facing away from the printed circuit board, and the sensor head may further comprise wire connections electrically connecting the contacts on the substrate to the printed circuit board.

Electrical signals may be formed at the plurality of sensors when the sensors make contact with the biological material for sensing one or more parameters. The electrical signals are conducted by the electrical conductors to the contacts and further via the wire connections to the printed circuit board.

Electrical signals may be conducted from the printed circuit board via the wire connections to the contacts and further via the electrical conductors to the sensors. This may be done by establishing a potential between electrodes of the sensors and measuring the current flowing through the electrodes of the sensors. Optionally, a current may be set up through the electrodes of the sensors and the voltage may be measured.

In one example, the sensors may be electrochemical sensors.

The printed circuit board may be provided with electronic circuitry for controlling the sensors and for processing the electrical signals received from the sensors. Thus, the printed circuit board may provide additional functionality of the sensor head. This implies that the substrate on which the sensors are arranged need not comprise any complex circuitry but may rather be used purely for transporting signals from the sensors to the contacts. This may facilitate use of the same type of substrate in a plurality of different applications. By manufacturing the substrate separately, the manufacturing process for manufacturing the substrate need not be changed when the sensors are to be used with different processing circuitry.

It should however be realized that more functionalities may be provided on the substrate on which the sensors are arranged such that the substrate need not be arranged on any printed circuit board.

According to one embodiment, the printed circuit board may comprise a connector, such as a zero-insertion force connector, for allowing an additional printed circuit board to be connected to the printed circuit board.

The additional printed circuit board may provide an electrical connection of the sensor head to devices external to the sensor head, such that the electrical signals may be transferred externally to the sensor head.

According to an embodiment, the second surface of the protective element may have an indentation for accommodating the wire connections between the substrate and the printed circuit board.

The indentation allows the wire connections to extend from the contacts on the substrate to the printed circuit board. An advantage of this is that the sensor head may have small dimensions, while yet allowing the wire connections to connect the contacts to the printed circuit board.

According to an embodiment, the opening through the protective element may have the dimensions of 5 cm to 0.2 mm, such as 5 cm, 3 cm, 10 mm to 1 mm, or such as 0.9 mm to 0.2 mm.

The dimensions may be length and width of the protective element, or it may be the radius of the protective element, depending on the shape of the protective element.

The opening through the protective element may have the dimensions of about 10 mm, or about 8 mm, or about 6 mm, or about 4 mm, or about 3 mm, or about 2 mm, or about 1 mm, or about 0.9 mm, or about 0.8 mm, or about 0.7 mm, or about 0.6 mm, or about 0.5 mm, or about 0.4 mm, or about 0.3 mm, or about 0.2 mm.

The dimensions may be length and width of the opening through the protective element, or it may be the radius of the opening through the protective element, depending on the shape of the opening through the protective element.

The opening through the protective element may have different shapes, such as being formed as a circle, triangle, or a rectangle, such as a square.

According to an embodiment, the cap may have the dimension of 1 mm to 10 cm, such as 1 mm to 10 mm, or such as 10 mm to 20 mm, or such as 20 mm to 30 mm, or such as 30 mm to 40 mm, or such as 40 mm to 50 mm, or such as 50 mm to 1 cm, or such as 1 cm to 10 cm.

The cap may have the dimensions of about 1 mm, or about 2 mm, or about 3 mm, or about 4 mm, or about 5 mm, or about 6 mm, or about 7 mm, or about 8 mm, or about 9 mm, or about 10 mm, or about 11 mm, or about 12 mm, or about 13 mm, or about 14 mm, or about 15 mm, or about 16 mm, or about 17 mm, or about 18 mm, or about 19 mm, or about 20 mm, or about 21 mm, or about 22 mm, or about 23 mm, or about 24 mm, or about 25 mm, or about 26 mm, or about 27 mm, or about 28 mm, or about 29 mm, or about 30 mm, or about 31 mm, or about 32 mm, or about 33 mm, or about 34 mm, or about 35 mm, or about 36 mm, or about 37 mm, or about 38 mm, or about 39 mm, or about 40 mm, or about 41 mm, or about 42 mm, or about 43 mm, or about 44 mm, or about 45 mm, or about 46 mm, or about 47 mm, or about 48 mm, or about 49 mm, or about 50 mm, or about 1 cm or about 10 cm.

The dimensions may be length and width of the cap, or it may be the radius of the cap, depending on the shape of cap.

The dimensions of the cap may be larger than the dimensions of the opening though the protective element.

The cap may have several different shapes, such as being a square or a circle.

The shape of the cap may be defined by the dimensions of the opening of the protective element and the wiring and sealing methods. The thickness of the cap is preferably minimized, so that the sensors easily may come directly in contact with the biological material.

According to an embodiment, the cap may comprise biocompatible materials, such as thermoplastics, such as polyetereterketon (PEEK), Poly(methyl methacrylate) (PMMA), Cyclic olefin copolymer (COC), Polycarbonate (PC), Polytetrafluoroethylene (PTFE).

The cap may be made by a biological compatible material.

According to a second aspect, there is provided a probe for insertion into a bioreactor, said probe comprising:
the sensor head according to the first aspect,
an elongate carrier, wherein the sensor head is mounted to a tip of the elongate carrier, the elongate carrier being configured to enclose the substrate arranged in a space inside the elongate carrier, wherein the sensor head is mounted to the elongate carrier to form a seal therebetween.

This aspect may generally present the same or corresponding advantages as the former aspect.

The sensor head may be mounted to a tip of the elongate carrier by the cap being sealed to the elongate carrier. For instance, the structure of the cap arranged at an outside of the area of the surface of the substrate may be sealed to the elongate carrier.

The probe may be configured for use inside of a bioreactor. It may further be configured for use inside any other biological container.

The probe may be configured to allow forming a seal at the entrance of the bioreactor or the biological container. Thus, contamination of the bioreactor or biological container may be prevented, while allowing the sensor head to be inserted into the bioreactor or the biological container.

The sensor head is mounted to the tip of the probe, such that the opening of the protective element of the sensor head exposes the plurality of sensors to the biological material surrounding the probe.

The sensor head is sealed to the elongate carrier such that the inside of the sensor head is protected against an environment external to the probe.

According to an embodiment, the surface of the substrate of the sensor head may be arranged at an angle of +/-90 ° to a longitudinal axis of the elongate carrier.

It should be understood that the longitudinal axis of the elongate carrier is an axis extending along the length of the elongate carrier.

The sensor head may be arranged at an angle of +/- 90° to the longitudinal axis, such as being arranged at an angle of 0°, 45° or 90°, or at an angle of -45° or -90° to the longitudinal angle.

The sensor head may be arranged at an angle of 30-60° to the longitudinal axis.

The arrangement of the sensor head to the longitudinal axis may be optimized for the specific use of the probe. As an example, the angle to the longitudinal axis may be used to prevent bubbles to form on the surface of the plurality of sensors. Such bubbles may prevent proper readout from the sensors.

According to an embodiment, the probe may further comprise the additional printed circuit board being connected to the connector of the printed circuit board of the sensor head, the additional printed circuit board being configured to extend along a longitudinal direction of the elongate carrier.

The additional printed circuit board may be a flexible printed circuit board.

A cross-section of the elongate carrier may be small. This implies that there is a small space at the tip of the elongate carrier in which a printed circuit board may be mounted. Thus, by having an additional printed circuit board extending along the longitudinal direction of the elongate carrier, the additional printed circuit board provides a relatively large area in which circuitry, e.g., for processing signals acquired by the sensors, may be arranged.

According to an embodiment, an end of the elongate carrier opposite to the tip at which the sensor head is mounted may be open to allow regulation of pressure of the space inside the elongate carrier.

An advantage of this is that it may prevent damages of the plurality of sensors in response to pressure changes due to for instance sterilization methods.

According to a third aspect, there is provided an sensing device comprising the sensor head according to the first aspect.

The sensing device may be an ingestible device.

This aspect may generally present the same or corresponding advantages as the former aspects.

The plurality of sensor may be used to measure parameters from inside of a body of a human or animal being, such as parameters from the stomach.

The sensor head may be mounted to a capsule adapted for being ingested. The sensor head may be mounted to the capsule by the cap being sealed to walls of the capsule. For instance, the structure of the cap arranged at an outside of the area of the surface of the substrate may be sealed to the walls of the capsule.

The walls of the capsule may be configured to enclose the substrate of the sensor head arranged in a space inside the walls of the capsule.

It should be understood that the small size of the sensor head is advantageous, since when the sensing device being an ingestible device, it should be very small for it to be ingestible. Further, the biocompatibility of the materials are advantageous, since the ingestible device must be non-toxic to the human or animal being.

According to a fourth aspect, there is provided a bioreactor comprising the sensor head according to the first aspect.

This aspect may generally present the same or corresponding advantages as the former aspects.

The sensor head may for instance be welded into an inner wall of a bag of a bioreactor or may be mounted within the bioreactor in another manner, exposing the sensors while preventing the substrate of the sensor head to come into contact with biological material in the bioreactor. The sensor head may be used to measure inside of the bioreactor. The sensor head may be connected to the outside of the bioreactor, through the bag of the bioreactor. Alternatively, the sensor head may be configured to wirelessly transmit signals to a device external to the bioreactor.

According to a fifth aspect, there is provided a flow cell comprising the sensor head according to the first aspect.

This aspect may generally present the same or corresponding advantages as the former aspects.

The sensor head may be mounted in a channel of a flow cell. Thus, the sensor head may be arranged such that the plurality of the sensors may be exposed to the flow inside of the flow cell, such that biological materials flowing in the channel may be exposed to the plurality of sensors.

The sensor head may be mounted at an inner wall of the channel of the flow cell, exposing the sensors while preventing the substrate of the sensor head to come into contact with biological material in the channel.

According to a sixth aspect, there is provided a method for manufacturing a sensor head for measurement of a biological material, the method comprising:
forming a plurality of sensors in a sensor area at a surface of a substrate;
forming electrical conductors between the plurality of sensors and contacts, wherein the electrical conductors are extending from the sensor area along the surface of the first substrate to the contacts;
attaching a cap to the substrate, wherein the cap comprises a protective element for providing protection from the biological material, the protective element comprising:
   a first surface and a second surface, opposite to the first surface;
   walls extending from the first surface to the second surface, defining an opening through the protective element;
wherein the attaching of the cap to the substrate comprises sealing the second surface of the protective element, around the walls of the protective element, to the surface of the substrate, at a position between the sensor area and the contacts, such that the opening of the protective element is arranged above the sensor area for allowing the plurality of sensors to be exposed to the biological material.

This aspect may generally present the same or corresponding advantages as the former aspects.

The methods form a plurality of sensors in a sensor area at the surface of the substrate.

By the sealing of the second surface of the protective element, around the walls of the protective element, to the surface of the substrate, the opening of the protective element, over the sensors, are provided while the inside of the sensor head is protected from the outside environment.

The sealing may be made by a high precision method, such that the sealing is made with high precision at a small size sensor head. The cap may present structures enabling sealing and mounting of other components to the sensor head with more lenient requirements on precision of mounting.

According to an embodiment, the method may further comprise
attaching a cover to the cap, wherein the cap further comprises a structure presenting a surface, wherein the protective element extends over an entire area of the surface of the substrate structure and the structure is arranged at an outside of the area of the surface of the substrate;
wherein the attaching of the cover to the cap comprises sealing the surface of the cap to the cover surrounding the substrate.

The sealing of the surface of the cap to the cover surrounding the substrate may be made with lower precision than the sealing of the protective element to the substrate.

The sealing of the surface of the cap to the cover surrounding the substrate enables the senor head to be fully sealed from the outside of the sensor head. Further, it enables arrangement of the sensor head to further structures, such as probes, ingestible devices, bioreactors or flow cells.

### Brief description of the drawings

The above, as well as additional objects, features, and advantages of the present description, will be better understood through the following illustrative and non-limiting detailed description, with reference to the appended drawings. In the drawings like reference numerals will be used for like elements unless stated otherwise.
Fig. 1a is a schematic view of a sensor head according to an embodiment of the present invention.
Fig. 1b is a schematic cross section view of a sensor head according to an embodiment of the present invention.
Fig. 2 is a schematic view of a sensor head according to an embodiment of the present invention.
Fig. 3a is a schematic view of a probe according to an embodiment of the present invention.
Fig. 3b is a schematic view of a probe according to an embodiment of the present invention.
Fig. 3c is a schematic view of a probe according to an embodiment of the present invention.
Fig. 3d is a schematic view of a probe according to an embodiment of the present invention.
Fig. 4 is a schematic view of a probe according to an embodiment of the present invention.
Fig. 5 is a schematic view of a sensing device according to an embodiment of the present invention.
Fig. 6 is a schematic view of a bioreactor according to an embodiment of the present invention.
Fig. 7 is a schematic view of a flow cell according to an embodiment of the present invention.
Fig. 8 is a schematic view of a method for manufacturing a sensor head according to an embodiment of the present invention.

### Detailed description

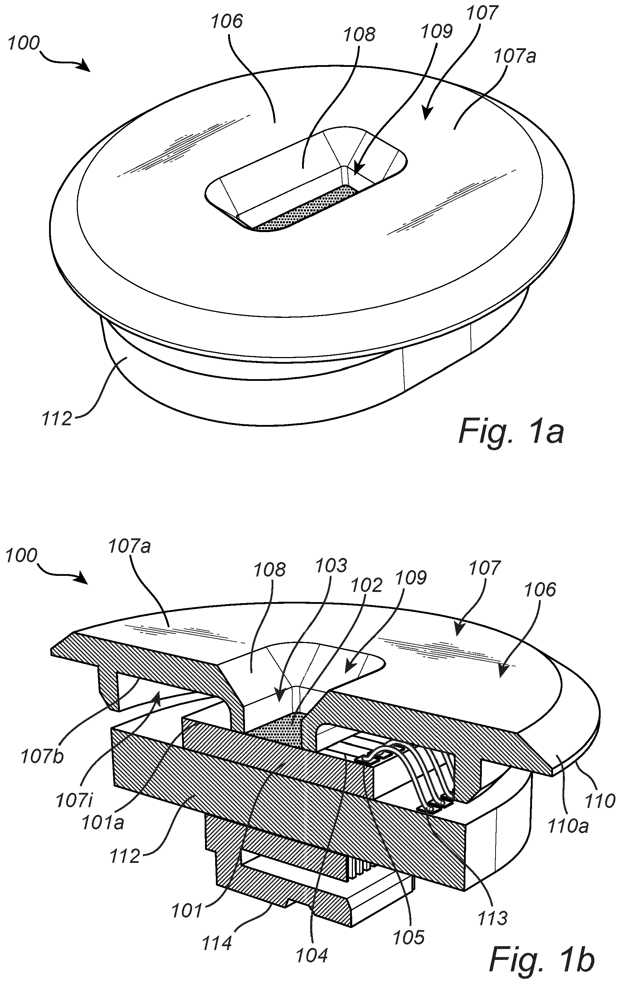

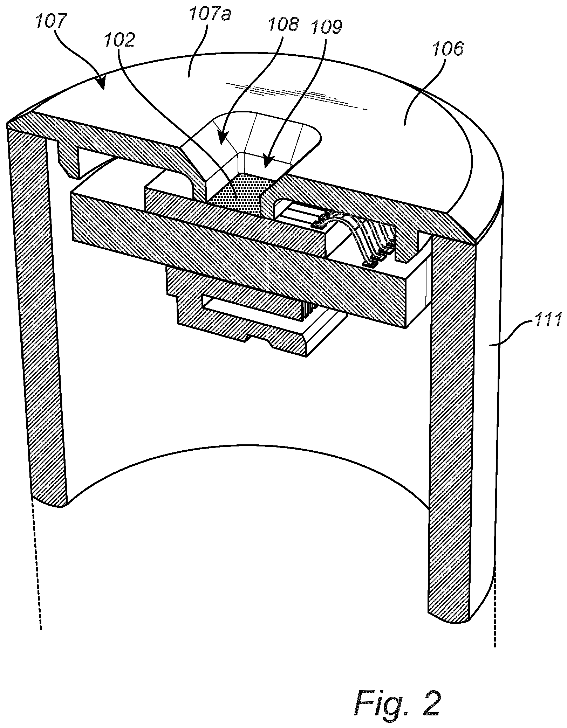

Figs 1a, 1b and 2 illustrate a sensor head 100 for measurement of a biological material.

The sensor head 100 comprises a substrate 101. The substrate 101 may be any substrate 101 on which sensors may be formed. The substrate 101 may for instance be a substrate 101 that is compatible with semiconductor processing, such as a silicon substrate, possibly provided with an insulating layer on the silicon substrate. The substrate 101 may alternatively be glass, which may be used as a substrate for forming structures by thin-film deposition on the substrate. The substrate 101 may for instance be a die, which may be formed by semiconductor processing. The substrate has a sensor area, at which the plurality of sensors is arranged.

The substrate 101 comprises a plurality of sensor 102 arranged in a sensor area 103 at a surface 101a of the substrate 101. The plurality of sensors 102 may comprise at least five sensors, such as at least 10 sensors or at least 20 sensors. In fact, the plurality of sensors may even comprise hundreds of sensors 102. Several of the plurality of sensors 102 may measure the same parameter. The plurality of sensors 102 may be configured to each measure a single parameter of the biological material. The plurality of sensors 102 may for instance measure glucose, lactate, electrical conductivity, dissolved oxygen, oxidation-reduction potential and cell density.

The plurality of sensors 102 may each be a conducting element configured for generating a signal after a stimulus. The plurality of sensors 102 may for instance comprise a noble metal, such as platinum or gold. The plurality of sensors 102 may further be functionalized such that the surface receiving the signal is more sensitive.

The sensor area 103 is arranged at a surface 101a of the substrate 101. The sensor area 103 is configured such that it can withstand exposure to biological material, which may be a liquid material.

The sensor head 100 further comprises electrical conductors 104. The electrical conductors 104 are connecting the plurality of sensors 102 to contacts 105. The electrical conductors 104 are extending from the sensor area 103, along the surface 101a of the substrate 101, to the contacts 105. By this, the electrical signals acquired by the plurality of the sensors 102 can be electrically conducted from the respective sensor 102 to the contacts 105.

The sensor head 100 further comprises a cap 106. The cap 106 comprises a protective element 107 for providing protection from the biological material. In other words, the protective element 107 ensures that the biological material may not reach an interior of the sensor head 100. By this, sensitive electrical parts of the sensor head 100 may be protected. The cap 106 may comprise biocompatible materials, such as thermoplastics, such as polyetereterketon (PEEK), Poly(methyl methacrylate) (PMMA), Cyclic olefin copolymer (COC), Polycarbonate (PC), Polytetrafluoroethylene (PTFE).

The cap 106 may have the dimension of 1 mm to 50 mm, such as 1 mm to 10 mm, or such as 10 mm to 20 mm, or such as 20 mm to 30 mm, or such as 30 mm to 40 mm, or such as 40 mm to 50 mm.

The cap 106 may have the dimensions of about 1 mm, or about 2 mm, or about 3 mm, or about 4 mm, or about 5 mm, or about 6 mm, or about 7 mm, or about 8 mm, or about 9 mm, or about 10 mm, or about 11 mm, or about 12 mm, or about 13 mm, or about 14 mm, or about 15 mm, or about 16 mm, or about 17 mm, or about 18 mm, or about 19 mm, or about 20 mm, or about 21 mm, or about 22 mm, or about 23 mm, or about 24 mm, or about 25 mm, or about 26 mm, or about 27 mm, or about 28 mm, or about 29 mm, or about 30 mm, or about 31 mm, or about 32 mm, or about 33 mm, or about 34 mm, or about 35 mm, or about 36 mm, or about 37 mm, or about 38 mm, or about 39 mm, or about 40 mm, or about 41 mm, or about 42 mm, or about 43 mm, or about 44 mm, or about 45 mm, or about 46 mm, or about 47 mm, or about 48 mm, or about 49 mm, or about 50 mm.

The dimensions of the cap 106 may be larger than the dimensions of the opening 109 through the protective element 107.

The cap 106 may have several different shapes, such as being a square or a circle. The shape of the cap 106 may be defined by the dimensions of the substrate 101 such that the entire substrate 101 may be covered by the cap 106. The thickness of the cap 106 is preferably minimized, so that the sensors 102 may easily make contact with the biological material.

The protective element 107 comprises a first surface 107a and a second surface 107b. The second surface 107b is opposite to the first surface 107a.

The protective element 107 further comprises walls 108 extending from the first surface 107a to the second surface 107b. The walls 108 define an opening 109 through the protective element 107.

The opening 109 through the protective element 107 may have the dimensions of 5 cm to 0.2 mm, such as 5 cm, 3 cm, 10 mm to 1 mm, or such as 0.9 mm to 0.2 mm.

The opening 109 through the protective element 107 may have the dimensions of about 5 cm, or about 3 cm or about 10 mm, or about 8 mm, or about 6 mm, or about 4 mm, or about 3 mm, or about 2 mm, or about 1 mm, or about 0.9 mm, or about 0.8 mm, or about 0.7 mm, or about 0.6 mm, or about 0.5 mm, or about 0.4 mm, or about 0.3 mm, or about 0.2 mm.

The opening 109 through the protective element 107 may have different shapes, such as being circle, or a square.

The second surface 107b of the protective element 107 is, around the walls 108 of the protective element 107, sealed to the surface 101a of the substrate 101 at a position between the sensor area 103 and the contacts 105 such that the opening 109 of the protective element 107 is arranged above the sensor area 103 for allowing the plurality of sensors 102 to be exposed to the biological material.

The sealing of the protective element 107 to the surface 101a of the substrate 101 may be made by the use of adhesives or by a physical bonding. The sealing may be a hermetical seal of the cap 106 to the substrate 101, such that only the sensor area 103 and the plurality of sensors 102 are exposed to the biological material.

The cap 106 may further comprise a structure 110. The structure 110 is presenting a surface 110a for allowing the cap 106 to be sealed to a cover 111 surrounding the substrate 101. The structure 110 may be a part of the protective element 107. However, it may also be a separate part of the cap 106. The cover 111 may have different shapes depending on the application in which the sensor head 100 is to be used.

It should be realized that the sensor head 100 and the cover 111 may be separately manufactured. The cover 111 may form part of a device in which the sensor head 100 is to be used. Thus, the cap 106 may be sealed to the cover 111 when the sensor head 100 is mounted in a device in which the sensor head 100 is to be used.

The protective element 107 extends over an entire area of the surface 101a of the substrate 101 and the structure 110 is arranged at an outside of the area of the surface 101a of the substrate 101. Thus, the protective element 107 and the structure 110 together cover the inside of the sensor head 100.

The sensor head 100 may further comprise a printed circuit board 112. The substrate 101 may then be attached to the printed circuit board 112 with the surface 101a of the substrate 101 on which the sensors 102 are arranged facing away from the printed circuit board 112. The sensor head 100 may further comprise wire connections 113 electrically connecting the contacts 105 on the substrate 101 to the printed circuit board 112.

The wire connections 113 may be used to transport the electrical signals from the contacts 105 to the printed circuit board 112. The printed circuit board 112 may be configured for allowing signals acquired by the sensors 102 to be transmitted to an external device outside of the sensor head 100. For instance, the printed circuit board 112 may comprise a communication unit for wireless communication with the external device.

The printed circuit board 112 may comprise a connector 114 for allowing an additional printed circuit board to be connected to the printed circuit board 112. The connector 114 may for instance be a zero-insertion force connector 114.

The second surface 107b of the protective element 107 may have an indentation 107i for accommodating the wire connections 113 between the substrate 101 and the printed circuit board 112. The indentation 107i may have different geometries and depth, such that it may be adapted for the specific configuration of wire connections 113, contacts 105 and printed circuit board 112.

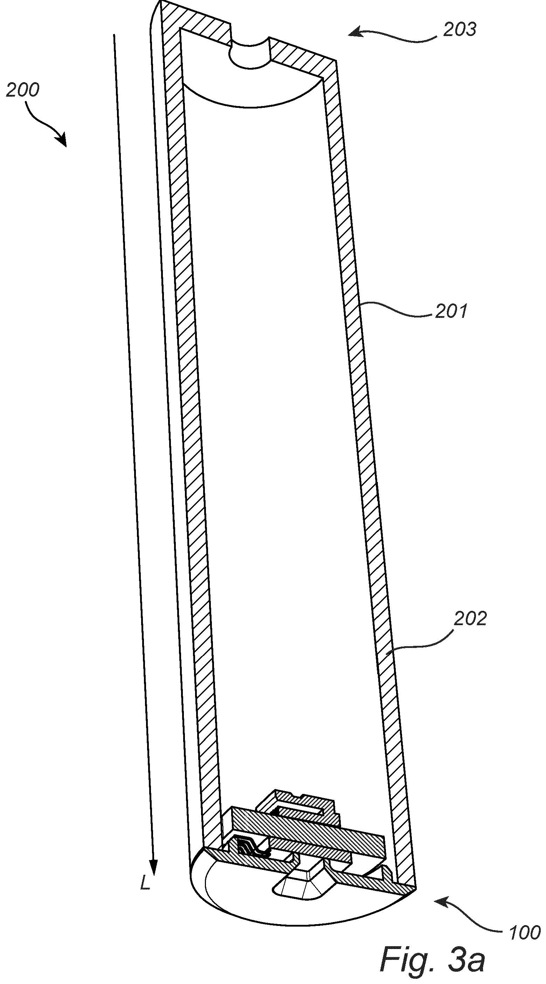

Fig. 3a illustrates a probe 200 for insertion into a bioreactor. The probe 200 comprises the sensor head 100 as described above.

The probe 200 further comprises an elongate carrier 201. The sensor head 100 is mounted to a tip 202 of the elongate carrier 201. The elongate carrier 201 is being configured to enclose the substrate 101 arranged in a space inside the elongate carrier 201. The sensor head 100 is mounted to the elongate carrier 201 to form a seal therebetween. Thus, when the sensor head 100 is used in a probe 200, the elongate carrier 201 may form the cover to which the sensor head 100 is sealed, as discussed above.

The probe 200 may be configured for use inside of a bioreactor. It may further be configured for use inside any other biological container.

The probe 200 may form a seal at the entrance of the bioreactor or the biological container.

The sensor head 100 is mounted to the tip 202 of the probe 200, such that the opening 109 of the protective element 107 of the sensor head 100 exposes the plurality of sensors 102 to the biological material surrounding the probe 200.

The sensor head 100 is sealed to the elongate carrier 201 such that the inside of the sensor head 100 is protected to the outside environment of the probe 200 and the sensor head 100.

As is illustrated in Fig. 3a-3d, the surface 101a of the substrate 101 of the sensor head 100 may be arranged at an angle of +/-90° to a longitudinal axis L of the elongate carrier 201. The surface 101a of the substrate 101 of the sensor head 100 may be arranged at an angle of 0°, 45° or 90°, or at an angle of -45° or -90° to the longitudinal axis L. Further, the angle to the longitudinal axis L may be 30-60°.

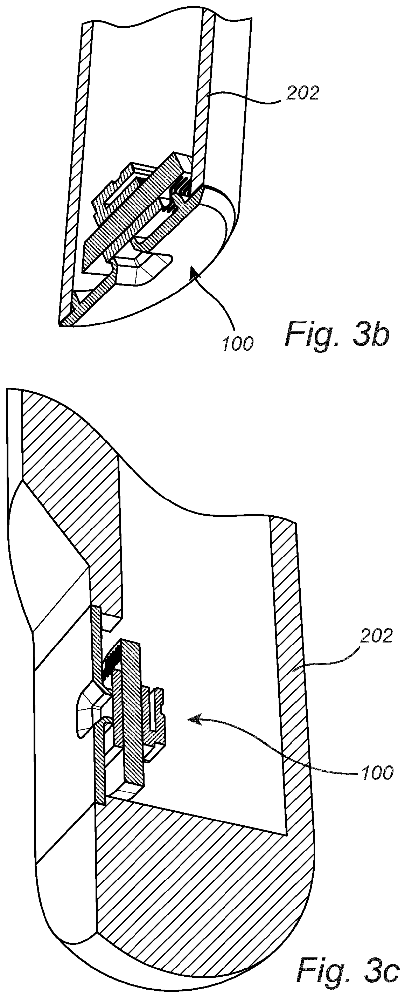

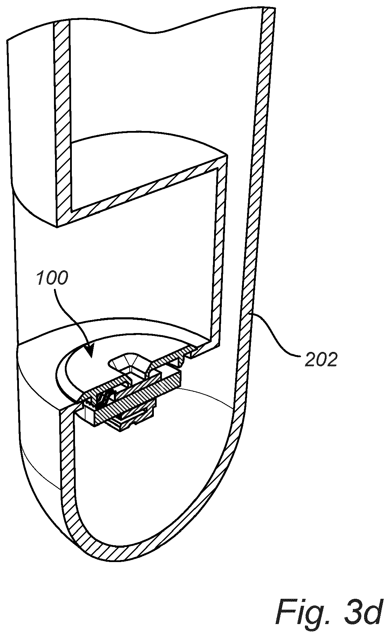

Fig. 3a illustrates the sensor head 100 arranged at an angle of 90° to the longitudinal axis L. Fig. 3b illustrates the sensor head 100 arranged at an angle of 45° to the longitudinal axis L. Fig. 3c illustrates the sensor head 100 arranged at an angle of 0° to the longitudinal axis L, Fig. 3d illustrates the sensor head 100 arranged at an angle of -90° to the longitudinal axis L.

An end 203 of the elongate carrier 201, opposite to the tip 202 at which the sensor head 100 is mounted, may be open to allow regulation of pressure of the space inside the elongate carrier 201.

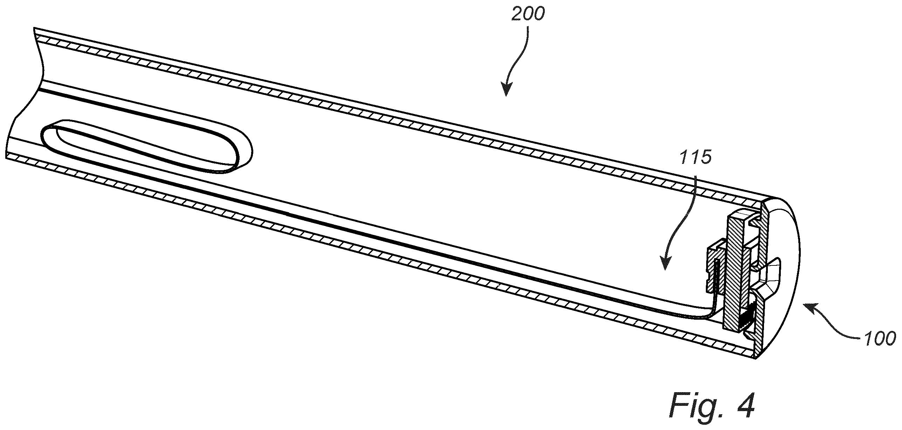

As is illustrated in Fig. 4, the probe 200 may further comprise the additional printed circuit board 115 being connected to the connector 114 of the printed circuit board 112 of the sensor head 100. The additional printed circuit board 115 may be configured to extend along a longitudinal direction L of the elongate carrier 201.

The additional printed circuit board 115 may be a flexible printed circuit board.

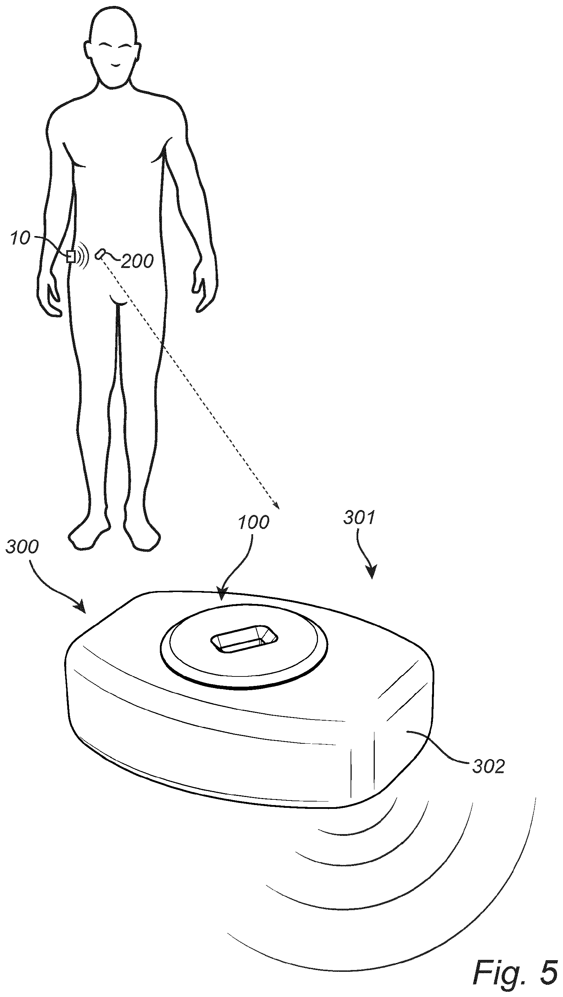

Fig. 5 illustrates a sensing device 300 comprising the sensor head 100. In one example, the sensing device 300 may be an ingestible or an implantable device. In the case of ingestible application, the sensing device 300 may be ingested and used for analysis of any compound or health status of the stomach.

The plurality of sensors 102 may be used to measure parameters from inside of a body of a human or animal being, such as parameters from the stomach.

The sensor head 100 may be mounted to a capsule 301 adapted for being ingested. The sensor head 100 may be mounted to the capsule 301 by the cap 106 being sealed to walls 302 of the capsule 301. For instance, the structure 110 of the cap 106 arranged at an outside of the area of the surface 110a of the substrate 101 may be sealed to the walls 302 of the capsule 301.

The walls 302 of the capsule 301 may be configured to enclose the substrate 101 of the sensor head 100 arranged in a space inside the walls 302 of the capsule 301.

The sensing device 300 may have different shapes such as being an ingestible device, such as a pill, The sensor head 100 may then have different shapes, such as following the curvature of a pill.

In one embodiment, the sensing device 300 may be positioned within a bioreactor container or bioreactor bag.

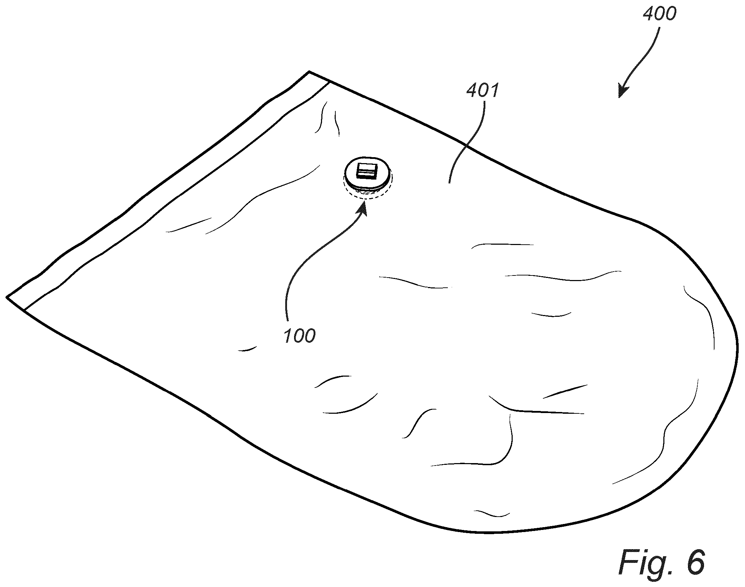

Fig. 6 illustrates a bioreactor 400 comprising the sensor head 100. The sensor head 100 may be sealed to a bag of a bioreactor 400. The sensor head 100 may be used to measure inside of the bioreactor 400. The sensor head 100 may be connected to the outside of the bioreactor, through the bag of the bioreactor 400.

In other words, the sensor head 100 may be sealed into the walls 401 of the bioreactor 400. The sensor head 100 may be mounted to the bioreactor 400 by the cap 106 being sealed to the walls 401 of the bioreactor. For instance, the cap 106 may be sealed or welded to the walls 401 of the bioreactor 400 such that parts of the sensor head 100 are exposed at the inside of the bioreactor, while the plurality of sensors 102 are exposed to the inside of the bioreactor 400.

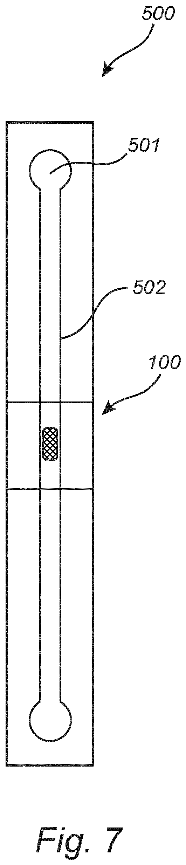

Fig. 7 illustrates a flow cell 500 comprising the sensor head 100. The sensor head 100 may be arranged into a channel 501 of a flow cell 500. Thus, the sensor head 100 may be arranged such that the plurality of the sensors 102 may be exposed to the flow inside of the flow cell 500, such that biological materials flowing in the channel 501 may be exposed to the plurality of sensors 102.

The sensor head 100 may be mounted at an inner wall 502 of the channel 501 of the flow cell, exposing the sensors 102 while preventing the substrate 101 of the sensor head 100 to come into contact with biological material in the channel 401.

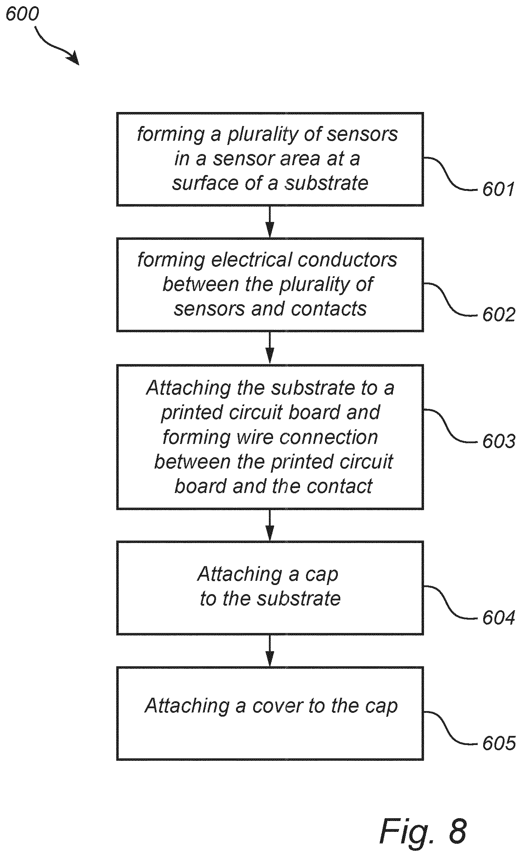

Fig. 8 illustrates a method 600 for manufacturing a sensor head 100 for measurement of a biological material.

The method 600 comprises forming 601 a plurality of sensors 102 in a sensor area 103 at a surface 101a of a substrate 101.

The method further comprises forming 602 electrical conductors 104 between the plurality of sensors 102 and contacts 105. The electrical conductors 104 are extending from the sensor area 103 along the surface 101a of the substrate 101 to the contacts 105.

The forming 601 a plurality of sensors 102 in a sensor area 103 at a surface 101a of a substrate 101 may be made simultaneously with forming 602 electrical conductors 104 between the plurality of sensors 102 and contacts 105.

Further, the method 600 may comprise attaching 603 the substrate 101 to a printed circuit board 112 and forming a wire connection 113 between the printed circuit board 112 and the and the contact 105.

The method 600 further comprises attaching 604 a cap 106 to the substrate 101. The cap 106 comprises a protective element 107 for providing protection from the biological material.

The protective element 107 comprises a first surface 107a and a second surface 107b. The second surface 107a being opposite to the first surface 107a.

The protective element 107 further comprises walls 108. The walls 108 extend from the first surface 107a to the second surface 107b and defines an opening 109 through the protective element 107.

The attaching 604 of the cap 106 to the substrate 101 comprises sealing the second surface 107b of the protective element 107, around the walls 108 of the protective element 107, to the surface 101a of the substrate 101, at a position between the sensor area 103 and the contacts 105, such that the opening 109 of the protective element 107 is arranged above the sensor area 103 for allowing the plurality of sensors 102 to be exposed to the biological material.

The attaching 604 of the cap 106 to the substrate 101 may be made by high-precision methods.

The attaching 604 may be made using adhesives or by physical sealing.

The attaching 604 of the cap 106 to the substrate 101 around the walls 108 of the protective element 107 provides aligning of the opening 109 of the protective element 107 over the plurality of sensors 102, such that only the sensors 102 are exposed to the biological materials.

The method 600 may further comprise attaching 605 a cover 111 to the cap 106. The cap 106 may further comprise a structure 110 presenting a surface 110a and the protective element 107 may extend over an entire area of the surface 101a of the substrate 101. The structure 110 may be arranged at an outside of the area of the surface 101a of the substrate 101.

The attaching 605 of the cover 111 to the cap 106 comprises sealing the surface 110a of the cap 106 to the cover 111 surrounding the substrate 101.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. A sensor head (100) for measurement of a biological material, the sensor head (100) comprising:
a substrate (101) comprising:
a plurality of sensors (102) arranged in a sensor area (103) at a surface (101a) of the substrate (101);
electrical conductors (104) connecting the plurality of sensors (102) to contacts (105), wherein the electrical conductors (104) are extending from the sensor area (103) along the surface (101a) of the substrate (101) to the contacts (105);
a cap (106) comprising a protective element (107) for providing protection from the biological material, the protective element (107) comprising:
a first surface (107a) and a second surface (107b), opposite to the first surface (107a);
walls (108) extending from the first surface (107a) to the second surface (107b), defining an opening (109) through the protective element (107);
wherein the second surface (107b) of the protective element (107), around the walls (108) of the protective element (107), is sealed to the surface (101a) of the substrate (101) at a position between the sensor area (103) and the contacts (105) such that the opening (109) of the protective element (107) is arranged above the sensor area (103) for allowing the plurality of sensors (102) to be exposed to the biological material.

2. The sensor head (100) according to claim 1, wherein the cap (106) further comprises a structure (110) presenting a surface (110a) for allowing the cap (106) to be sealed to a cover (111) surrounding the substrate (101), wherein the protective element (107) extends over an entire area of the surface (101a) of the substrate (101) and the structure (110) is arranged at an outside of the area of the surface (101a) of the substrate (101).

3. The sensor head (100) according to claims 1 or 2, further comprising a printed circuit board (112), wherein the substrate (101) is attached to the printed circuit board (112) with the surface (101a) of the substrate (101) on which the sensors (102) are arranged facing away from the printed circuit board (112), the sensor head (100) further comprising wire connections (113) electrically connecting the contacts (105) on the substrate (101) to the printed circuit board (112).

4. The sensor head (100) according to claim 3, wherein the second surface (107b) of the protective element (107) has an indentation (107i) for accommodating the wire connections (113) between the substrate (101) and the printed circuit board (112).

5. The sensor head (100) according to any one of claims 1 to 4, wherein the opening (109) through the protective element (107) has dimensions of 5 cm to 0.2 mm.

6. The sensor head (100) according to any one of claims 1 to 5, wherein the cap (106) has the dimension of 1 mm to 10 cm.

7. The sensor head (100) according to any one of claims 1 to 6, wherein the cap (106) comprises biocompatible materials, such as thermoplastics, such as polyetereterketon (PEEK), Poly(methyl methacrylate) (PMMA), Cyclic olefin copolymer (COC), Polycarbonate (PC), Polytetrafluoroethylene (PTFE).

8. A probe (200) for insertion into a bioreactor, said probe comprising:
the sensor head (100) according to any one of claims 1 to 7,
an elongate carrier (201), wherein the sensor head (100) is mounted to a tip (202) of the elongate carrier (201), the elongate carrier (201) being configured to enclose the substrate (101) arranged in a space inside the elongate carrier (201), wherein the sensor head (100) is mounted to the elongate carrier (201) to form a seal therebetween.

9. The probe (200) according to claim 8, wherein the surface (101a) of the substrate (101) of the sensor head (100) is arranged at an angle of +/-90 ° to a longitudinal axis (L) of the elongate carrier (201).

10. The probe (200) according to claim 9, wherein an end (203) of the elongate carrier (201) opposite to the tip (202) at which the sensor head (100) is mounted is open to allow regulation of pressure of the space inside the elongate carrier (201).

11. A sensing device (300) comprising the sensor head (100) according to any one of claims 1 to 7.

12. A bioreactor (400) comprising the sensor head according to any one of claims 1 to 7.

13. A flow cell (500) comprising the sensor head according to any one of claims 1 to 7.

14. A method (600) for manufacturing a sensor head (100) for measurement of a biological material, the method (600) comprising:
forming (601) a plurality of sensors (102) in a sensor area (103) at a surface (101a) of a substrate (101);
forming (602) electrical conductors (104) between the plurality of sensors (102) and contacts (105), wherein the electrical conductors (104) are extending from the sensor area (103) along the surface (101a) of the substrate (101) to the contacts (105);
attaching (603) a cap (106) to the substrate (101), wherein the cap (106) comprises a protective element (107) for providing protection from the biological material, the protective element (107) comprising:
a first surface (107a) and a second surface (107b), opposite to the first surface (107a);
walls (108) extending from the first surface (107a) to the second surface (107b), defining an opening (109) through the protective element (107);
wherein the attaching (603) of the cap (106) to the substrate (101) comprises sealing the second surface (107b) of the protective element (107), around the walls (108) of the protective element (107), to the surface (101a) of the substrate (101), at a position between the sensor area (103) and the contacts (105), such that the opening (109) of the protective element (107) is arranged above the sensor area (103) for allowing the plurality of sensors (102) to be exposed to the biological material.

15. The method (600) according to claim 14, wherein the method (600) further comprises
attaching (604) a cover (111) to the cap (106), wherein the cap (106) further comprises a structure (110) presenting a surface (110a), wherein the protective element (107) extends over an entire area of the surface (101a) of the substrate (101) and the structure (110) is arranged at an outside of the area of the surface (101a) of the substrate (101);
wherein the attaching (604) of the cover (111) to the cap (106) comprises sealing the surface (110a) of the cap (106) to the cover (111) surrounding the substrate (101).

## Patentansprüche

1. Sensorkopf (100) zum Messen eines biologischen Materials, wobei der Sensorkopf (100) umfasst:
ein Substrat (101) umfassend:
eine Vielzahl von Sensoren (102), die in einem Sensorbereich (103) an einer Oberfläche (101a) des Substrats (101) angeordnet sind;
elektrische Leiter (104), welche die Vielzahl von Sensoren (102) mit Kontakten (105) verbinden, wobei sich die elektrischen Leiter (104) von dem Sensorbereich (103) entlang der Oberfläche (101a) des Substrats (101) bis zu den Kontakten (105) erstrecken;
eine Kappe (106), die ein Schutzelement (107) umfasst, um Schutz vor dem biologischen Material bereitzustellen, wobei das Schutzelement (107) umfasst:
eine erste Oberfläche (107a) und eine zweite Oberfläche (107b), gegenüber der ersten Oberfläche (107a);
Wände (108), die sich von der ersten Oberfläche (107a) bis zu der zweiten Oberfläche (107b) erstrecken und eine Öffnung (109) durch das Schutzelement (107) hindurch definieren;
wobei die zweite Oberfläche (107b) des Schutzelements (107) um die Wände (108) des Schutzelements (107) herum an der Oberfläche (101a) des Substrats (101) in einer Position zwischen dem Sensorbereich (103) und den Kontakten (105) versiegelt ist, so dass die Öffnung (109) des Schutzelements (107) über dem Sensorbereich (103) angeordnet ist, damit die Vielzahl von Sensoren (102) dem biologischen Material ausgesetzt werden kann.

2. Sensorkopf (100) nach Anspruch 1, wobei die Kappe (106) ferner eine Struktur (110) umfasst, die eine Oberfläche (110a) aufweist, damit die Kappe (106) an einer Abdeckung (111), die das Substrat (101) umgibt, versiegelt werden kann, wobei sich das Schutzelement (107) über einen gesamten Bereich der Oberfläche (101a) des Substrats (101) erstreckt, und die Struktur (110) außerhalb des Bereichs der Oberfläche (101a) des Substrats (101) angeordnet ist.

3. Sensorkopf (100) nach Anspruch 1 oder 2, ferner umfassend eine Leiterplatte (112), wobei das Substrat (101) an der Leiterplatte (112) angebracht ist, wobei die Oberfläche (101a) des Substrats (101), auf der die Sensoren (102) angeordnet sind, von der Leiterplatte (112) abgewandt ist, wobei der Sensorkopf (100) ferner Drahtverbindungen (113) umfasst, welche die Kontakte (105) auf dem Substrat (101) mit der Leiterplatte (112) elektrisch verbinden.

4. Sensorkopf (100) nach Anspruch 3, wobei die zweite Oberfläche (107b) des Schutzelements (107) eine Einkerbung (107i) aufweist, um die Drahtverbindungen (113) zwischen dem Substrat (101) und der Leiterplatte (112) aufzunehmen.

5. Sensorkopf (100) nach einem der Ansprüche 1 bis 4, wobei die Öffnung (109) durch das Schutzelement (107) hindurch Abmessungen von 5 cm bis 0,2 mm aufweist.

6. Sensorkopf (100) nach einem der Ansprüche 1 bis 5, wobei die Kappe (106) Abmessungen von 1 mm bis 10 cm aufweist.

7. Sensorkopf (100) nach einem der Ansprüche 1 bis 6, wobei die Kappe (106) körperverträgliche Materialen umfasst, wie etwa Thermoplaste, wie etwa Polyetheretherketon (PEEK), Polymethylmethacrylat (PMMA), Cycloolefin-Copolymer (COC), Polycarbonat (PC), Polytetrafluorethylen (PTFE).

8. Sonde (200) zum Einsetzen in einen Bioreaktor, wobei die Sonde umfasst:
den Sensorkopf (100) nach einem der Ansprüche 1 bis 7,
einen länglichen Träger (201), wobei der Sensorkopf (100) an einer Spitze (202) des länglichen Trägers (201) montiert ist, wobei der längliche Träger (201) dazu konfiguriert ist, das Substrat (101) zu umschließen, das in einem Raum im Innern des länglichen Trägers (201) angeordnet ist, wobei der Sensorkopf (100) an dem länglichen Träger (201) montiert ist, um eine Dichtung dazwischen zu bilden.

9. Sonde (200) nach Anspruch 8, wobei die Oberfläche (101a) des Substrats (101) des Sensorkopfs (100) in einem Winkel von +/-90° zu einer Längsachse (L) des länglichen Trägers (201) angeordnet ist.

10. Sonde (200) nach Anspruch 9, wobei ein Ende (203) des länglichen Trägers (201) gegenüber der Spitze (202), an welcher der Sensorkopf (100) montiert ist, offen ist, um das Regulieren des Drucks des Raums im Innern des länglichen Trägers (201) zu ermöglichen.

11. Sensorvorrichtung (300), umfassend den Sensorkopf (100) nach einem der Ansprüche 1 bis 7.

12. Bioreaktor (400), umfassend den Sensorkopf nach einem der Ansprüche 1 bis 7.

13. Flusszelle (500), umfassend den Sensorkopf nach einem der Ansprüche 1 bis 7.

14. Verfahren (600) zum Herstellen eines Sensorkopfes (100) zum Messen eines biologischen Materials, wobei das Verfahren (600) umfasst:
Bilden (601) einer Vielzahl von Sensoren (102) in einem Sensorbereich (103) an einer Oberfläche (101a) eines Substrats (101);
Bilden (602) von elektrischen Leitern (104) zwischen der Vielzahl von Sensoren (102) und Kontakten (105), wobei sich die elektrischen Leiter (104) von dem Sensorbereich (103) entlang der Oberfläche (101a) des Substrats (101) bis zu den Kontakten (105) erstrecken;
Anbringen (603) einer Kappe (106) an dem Substrat (101), wobei die Kappe 20 (106) ein Schutzelement (107) umfasst, um Schutz vor dem biologischen Material bereitzustellen, wobei das Schutzelement (107) umfasst:
eine erste Oberfläche (107a) und eine zweite Oberfläche (107b), gegenüber der ersten Oberfläche (107a);
Wände (108), die sich von der ersten Oberfläche (107a) bis zu der zweiten Oberfläche (107b) erstrecken und eine Öffnung (109) durch das Schutzelement (107) hindurch definieren;
wobei das Anbringen (603) der Kappe (106) an dem Substrat (101) das Versiegeln der zweiten Oberfläche (107b) des Schutzelements (107) um die Wände (108) des Schutzelements (107) herum an der Oberfläche (101a) des Substrats (101) in einer Position zwischen dem Sensorbereich (103) und den Kontakten (105) umfasst, so dass die Öffnung (109) des Schutzelements (107) über dem Sensorbereich (103) angeordnet ist, damit die Vielzahl von Sensoren (102) dem biologischen Material ausgesetzt werden kann.

15. Verfahren (600) nach Anspruch 14, wobei das Verfahren (600) ferner umfasst:
Anbringen (604) einer Abdeckung (111) an der Kappe (106), wobei die Kappe (106) ferner eine Struktur (110) umfasst, die eine Oberfläche (110a) aufweist, wobei sich das Schutzelement (107) über einen gesamten Bereich (101a) des Substrats (101) erstreckt, und die Struktur (110) außerhalb des Bereichs der Oberfläche (101a) des Substrats (101) angeordnet ist;
wobei das Anbringen (604) der Abdeckung (111) an der Kappe (106) das Versiegeln der Oberfläche (110a) der Kappe (106) an der Abdeckung (111), die das Substrat (101) umgibt, umfasst.

## Revendications

1. Tête de capteur (100) pour la mesure d'un matériau biologique, la tête de capteur (100) comprenant :
un substrat (101) comprenant :
une pluralité de capteurs (102) disposés dans une zone de capteur (103) au niveau d'une surface (101a) du substrat (101) ;
des conducteurs électriques (104) connectant les capteurs (102) à des contacts (105), dans laquelle les conducteurs électriques (104) s'étendent de la zone de capteur (103) le long de la surface (101a) du substrat (101) jusqu'aux contacts (105) ;
un capuchon (106) comprenant un élément protecteur (107) destiné à protéger contre le matériau biologique, l'élément protecteur (107) comprenant :
une première surface (107a) et une deuxième surface (107b), opposée à la première surface (107a) ;
des parois (108) s'étendant de la première surface (107a) à la deuxième surface (107b), définissant une ouverture (109) à travers l'élément de protection (107) ;
dans laquelle la deuxième surface (107b) de l'élément de protection (107), autour des parois (108) de l'élément de protection (107), est scellée à la surface (101a) du substrat (101) à un emplacement situé entre la zone de capteur (103) et les contacts (105), de sorte que l'ouverture (109) de l'élément de protection (107) soit disposée au-dessus de la zone de capteur (103) pour permettre l'exposition de la pluralité de capteurs (102) au matériau biologique.

2. Tête de capteur (100) selon la revendication 1, dans laquelle le capuchon (106) comprend en outre une structure (110) présentant une surface (110a) permettant au capuchon (106) d'être scellé à un couvercle (111) entourant le substrat (101), dans laquelle l'élément de protection (107) s'étend sur toute la surface (101a) du substrat (101) et la structure (110) est disposée à l'extérieur de la surface (101a) du substrat (101).

3. Tête de capteur (100) selon les revendications 1 ou 2, comprenant en outre une carte de circuit imprimé (112), dans laquelle le substrat (101) est fixé à la carte de circuit imprimé (112) de sorte que la surface (101a) du substrat (101) sur laquelle sont disposés les capteurs (102) soit orientée à l'opposé de la carte de circuit imprimé (112), la tête de capteur (100) comprenant en outre des connexions filaires (113) connectant électriquement les contacts (105) sur le substrat (101) à la carte de circuit imprimé (112).

4. Tête de capteur (100) selon la revendication 3, dans laquelle la deuxième surface (107b) de l'élément de protection (107) présente une indentation (107i) destinée à recevoir les connexions filaires (113) entre le substrat (101) et la carte de circuit imprimé (112).

5. Tête de capteur (100) selon une quelconque des revendications 1 à 4, dans laquelle l'ouverture (109) à travers l'élément de protection (107) a des dimensions de 5 cm à 0,2 mm.

6. Tête de capteur (100) selon une quelconque des revendications 1 à 5, dans laquelle le capuchon (106) a une dimension de 1 mm à 10 cm.

7. Tête de capteur (100) selon une quelconque des revendications 1 à 6, dans laquelle le capuchon (106) comprend des matériaux biocompatibles, tels que des thermoplastiques, tels que le polyéthercétone (PEEK), le polyméthacrylate de méthyle (PMMA), le copolymère d'oléfine cyclique (COC), le polycarbonate (PC), le polytétrafluoroéthylène (PTFE).

8. Sonde (200) destinée à être insérée dans un bioréacteur, ladite sonde comprenant :
la tête de capteur (100) selon une quelconque des revendications 1 à 7,
un support allongé (201), dans laquelle la tête de capteur (100) est montée sur une pointe (202) du support allongé (201), le support allongé (101) étant configuré pour envelopper le substrat (101) disposé dans un espace à l'intérieur du support allongé (201), dans laquelle la tête de capteur (100) est montée sur le support allongé (201) de manière à assurer l'étanchéité entre les deux.

9. Sonde (200) selon la revendication 8, dans laquelle la surface (101a) du substrat (101) de la tête de capteur (100) est disposée à un angle de +/-90° par rapport à un axe longitudinal (L) du support allongé (201).

10. Sonde (200) selon la revendication 9, dans laquelle une extrémité (203) du support allongé (201) opposée à la pointe (202), à laquelle la tête de capteur (100) est montée, est ouverte afin de permettre la régulation de la pression de l'espace à l'intérieur du support allongé (201).

11. Dispositif de détection (300) comprenant la tête de capteur (100) selon une quelconque des revendications 1 à 7.

12. Bioréacteur (400) comprenant la tête de capteur selon une quelconque des revendications 1 à 7.

13. Cellule à flux (500) comprenant la tête de capteur selon une quelconque des revendications 1 à 7.

14. Procédé (600) de fabrication d'une tête de capteur (100) pour la mesure d'un matériau biologique, le procédé (600) comprenant :
la formation (601) d'une pluralité de capteurs (102) dans une zone de capteur (103) à une surface (101a) d'un substrat (101) ;
la formation (602) de conducteurs électriques (104) entre la pluralité de capteurs (102) et les contacts (105), dans lequel lesdits conducteurs électriques (104) s'étendent de la zone de capteur (103) le long de la surface (101a) du substrat (101) aux contacts (105) ;
la fixation (603) d'un capuchon (106) sur le substrat (101), dans lequel le capuchon (106) comprend un élément protecteur (107) assurant la protection contre le matériel biologique, l'élément de protection (107) comprenant :
une première surface (107a) et une deuxième surface (107b), opposée à la première surface (107a) ;
des parois (108) s'étendant de la première surface (107a) à la deuxième surface (107b), définissant une ouverture (109) à travers l'élément de protection (107) ;
dans lequel la fixation (603) du capuchon (106) au substrat (101) consiste à sceller la deuxième surface (107b) de l'élément de protection (107), autour des parois (108) de l'élément de protection (107), à la surface (101a) du substrat (101), à un emplacement situé entre la zone de capteur (103) et les contacts (105), de sorte que l'ouverture (109) de l'élément de protection (107) soit disposée au-dessus de la zone de capteur (103) pour permettre l'exposition des capteurs (102) au matériau biologique.

15. Procédé (600) selon la revendication 14, dans lequel le procédé (600) comprend en outre :
la fixation (604) d'un couvercle (111) sur le capuchon (106), dans lequel le capuchon (106) comprend en outre une structure (110) présentant une surface (110a), dans lequel l'élément protecteur (107) s'étend sur toute la surface (101a) du substrat (101) et la structure (110) est disposée à l'extérieur de la zone de la surface (101a) du substrat (101) ;
dans lequel la fixation (604) du couvercle (111) sur le capuchon (106) consiste à sceller la surface (110a) du capuchon (106) au couvercle (111) entourant le substrat (101).
